# EUROPÄISCHE PATENTSCHRIFT

(11) **EP 1 825 817 B1**
(45) Veröffentlichungstag und Bekanntmachung des Hinweises auf die Patenterteilung: **21.07.2010**
(21) Anmeldenummer: 07003623.1
(22) Anmeldetag: 22.02.2007
(51) Int. Cl.: A61B 17/04

(54) **Ankerelement zum knotenfreien Fixieren von Gewebe an einem Knochen**
Anchor element for knotless attachment of tissue to a bone
Elément d'ancrage destiné à la fixation sans noeud de tissu sur un os

(30) Priorität: 27.02.2006 DE 102006010116
(43) Veröffentlichungstag der Anmeldung: 29.08.2007
(73) Patentinhaber: Karl Storz GmbH & Co. KG, 78532 Tuttlingen (DE); Universität Zürich, 8006 Zürich (CH)
(72) Erfinder: Gerber, Christian, 8126 Zumikon (CH); Meyer, Dominik, Dr., 8008 Zürich (CH); Berberich, Sascha, 78532 Tuttlingen (DE)
(74) Vertreter: Weller, Wolfgang

(56) Entgegenhaltungen:
- US-A- 5 464 427
- US-A- 5 707 395
- US-A1- 2005 055 052
- US-A1- 2005 245 932

## Beschreibung

Die Erfindung betrifft ein Ankerelement zum knotenfreien Fixieren von Gewebe an einem Knochen mittels zumindest eines durch das Ankerelement hindurchgefädelten Fadens, mit einem Körper, an dessen Außenseite Vorsprünge vorgesehen sind, die ein Abziehen des im Knochen eingebrachten Ankerelementes hindern, mit einer im distalen Endbereich des Körpers angeordneten und durch diesen hindurchreichenden Querbohrung zum Durchfädeln des zumindest einen Fadens quer durch den Körper hindurch, und mit einem längs des Körpers bewegbaren Klemmelementes zum Klemmen des durch den Körper hindurchgefädelten Fadens, wobei das Klemmelement innerhalb des Körpers angeordnet ist und zum Klemmen des Fadens in Richtung der Querbohrung bewegbar ist.

Ein derartiges Ankerelement ist beispielsweise aus der US 2004/0138706 A1 bekannt.

Solche Ankerelemente, auch Fadenanker genannt, werden im medizinischen Bereich dazu eingesetzt, um von einem Knochen abgelöstes Gewebe, meist Sehnen, wieder am Knochen zu fixieren.

Grundsätzlich wird dazu das Ankerelement samt einem mit dem Ankerelement verbundenen Faden in den Knochen festsitzend eingetrieben. Die vorstehenden Fadenenden werden mit dem abgelösten Gewebe verbunden, dadurch wird das losgelöste Gewebe am Knochen fixiert.

Bei einer ersten, insbesondere aus der US 5,690,676 bekannten Operationstechnik ist das Ankerelement so ausgestaltet, dass es einen etwa zylindrischen Körper aufweist, an dessen Außenseite Vorsprünge vorgesehen sind, die ein Abziehen des Ankerelements, nachdem es in den Knochen eingebracht worden ist, hindern. Diese Vorsprünge können als widerhakenartige Elemente ausgebildet sein, wenn beispielsweise das Ankerelement in den Knochen eingeschlagen oder eingetrieben wird oder können auch als Außengewinde ausgebildet sein, wenn das Ankerelement in Art einer Schraube in den Knochen eingedreht wird. Der Faden ist dabei durch die durch den Körper hindurchreichende Querbohrung durchgefädelt und die beiden Fadenenden sind während des Eintreibvorganges in äußeren Längsnuten am Körper eingelegt und nach proximal geführt. Am proximalen Ende des Ankerelementes greift ein sogenannter Eintreiber an, der üblicherweise auf das proximale Ende des Ankerelementes aufgesetzt wird. Die beiden Fadenenden werden längs des Eintreibers geführt und dort an radial vorstehenden Zapfen für den Eintreibvorgang aufgewickelt.

Nachdem das Ankerelement in den Knochen eingetrieben worden ist und der Eintreiber abgenommen ist, dienen die beiden freien Fadenenden dazu, das abgelöste Gewebe zu befestigen. Dazu werden die beiden Fadenenden mit dem abgelösten Gewebe, beispielsweise mit einer Sehne, eng am Knochen anliegend verknotet.

Das im Knochen verankerte Ankerelement einerseits und der Knoten andererseits bilden die Kraft/Widerlagerpunkte, zwischen denen das Gewebe fixiert ist.

Nachteilig an dieser Operationstechnik ist, dass das Aufknoten eine erhebliche Erfahrung und Fingerfertigkeit des Operateurs erfordert. Derartige Knoten können sich lösen, oder es können sich, da der Knoten von der Außenseite her eingebracht werden muss, Weichteilbrücken bilden.

In einer Weiterentwicklung dieser Operationstechnik wurden sogenannte knotenfreie Anker entwickelt, wie sie beispielsweise aus US 2004/0138706 A1 bekannt sind und die auch Gegenstand der vorliegenden Erfindung sind.

Auch bei dieser Operationstechnik wird der Faden zunächst durch das Ankerelement hindurchgefädelt. Eines der freien Fadenenden wird, meist unter Zuhilfenahme einer Nadel, durch das zu fixierende Gewebe hindurchgestochen und das durchgestochene Ende wird wieder in entgegengesetzter Richtung durch die Querbohrung im Ankerelement hindurchgefädelt. Die Verbindung zwischen Ankerelement und zu fixierendem Gewebe besteht in Form einer Fadenschleife. Das Ankerelement kann schon jetzt samt Faden in den Knochen eingebracht werden und es wird anschließend an den freien Fadenenden gezogen, wodurch die vorstehende Schleife des Fadens, die mit dem Gewebe verbunden ist, an die zu fixierende Stelle herangezogen wird.

Zum Fixieren der Relativstellung zwischen Faden und damit verbundenem Gewebe und Ankerelement wird nunmehr kein Knoten bewerkstelligt, sondern es wird ein Klemmelement bewegt, durch das der Faden in einer bestimmten Position am Ankerelement fixiert sprich geklemmt wird. Dadurch wird die Schleife, die das Gewebe hält, ebenfalls fixiert. Die überstehenden freien Enden können dann beispielsweise abgeschnitten werden und es ist nicht notwendig, einen Knoten anzubringen.

Bei der US 2004/0138706 A1 ist das Klemmelement als eine auf der Außenseite des Körpers des Ankerelements aufgebrachte Hülse ausgebildet. Hülse und Körper sind relativ zueinander verschiebbar.

In einer Schiebestellung der Hülse ist der durch den Körper hindurchgefädelte Faden frei beweglich, beispielsweise um das vom Faden durchstochene Gewebe an den Knochen heranzuziehen und in seiner Position zu bestimmen. Anschließend wird die Hülse verschoben, um dadurch den Faden zu verklemmen und in seiner Relativposition zu fixieren.

Wie insbesondere aus dem Übergang von Figur 4 zu Figur 5 der US 2004/0138706 A1 ersichtlich ist, bestehen mehrere relativ scharfkantige Klemmstellen, zwischen denen der Faden eingequetscht ist. Dadurch treten relativ hohe Scherkräfte auf, so dass eine Verletzung des Fadens und somit ein Abreißen nicht mit Sicherheit ausgeschlossen werden kann.

Darüber hinaus ist die äußere Hülse ein sehr kompliziert geformtes Bauteil, das, zum Ausüben einer Klemmkraft, durch das Ankerelement etwas gespreizt werden muss. Dazu sind entsprechende rastartige Übergänge zwischen Außenseite des Körpers des Ankerelementes und der Innenseite der Hülse notwendig, die ein Lösen dieser Rastposition erschweren oder unmöglich machen. Somit sind Korrekturen, beispielsweise unter einem zeitweiligen Lösen der klemmenden Verbindung, kaum oder nur schwierig durchzuführen.

Dabei ist zu bedenken, dass die Maße solcher Klemmelemente im Bereich von Längen von einigen Zentimetern und Durchmessern von einigen Millimetern liegen.

Daher ist nicht nur die Fertigung solcher Teile äußerst aufwändig, sondern auch deren Handhabung ist sehr schwierig und insbesondere ist deren Stabilität bezüglich der Halte- oder Fixierkraft äußerst problematisch.

Wird eine hochbeanspruchte Sehne, beispielsweise aus dem Schulterbereich oder dem Kniebereich, fixiert, so ist einleuchtend, dass erhebliche Zugkräfte von der Sehne auf den im Knochen eingebrachten Zusammenbau aus Körper, Klemmelement und dazwischen geklemmten Faden einwirken.

Bedenkt man die zuvor erwähnten Baugrößen, so ist einleuchtend, dass die Wandstärke der äußeren Hülse allenfalls im Bereich von Bruchteilen von Millimetern liegen kann, wobei aber gerade dieses Bauteil die Klemmkraft zum Halten des Fadens bereitstellen soll.

Da die Hülse bauartbedingt einen gewissen Anteil der Außenseite des Körpers des Ankerelementes belegt, dieses Ankerelement aber dazu dient, über die an seiner Außenseite vorhandenen Vorsprünge den gesamten Zusammenbau im Knochen zu halten, müssen entsprechende konstruktive Vorkehrungen getroffen werden, damit der Körper des Ankerelements als solcher überhaupt sicher im Knochen verankert werden kann.

Dies führt nun zu weiterem erheblich konstruktivem Aufwand.

Aus der US 2005/0055052 A1 ist ein knotenfreies Ankerelement bekannt, das ein an einem proximalen Ende des Ankerelements angeordnetes inneres Element aufweist. Das innere Element weist zwei Querbohrungen zum Durchfädeln von chirurgischen Fäden auf. Ferner ist das Ankerelement mit einem äußeren als Hülse ausgebildeten Element vorgesehen, das ebenfalls Querbohrungen aufweist, und auf dem inneren Element verschiebbar angeordnet ist. Um den durch die Querbohrungen des inneren Elements durchgefädelten Faden in den Querbohrungen zu klemmen, wird zuerst das äußere Element derart an das innere Element angebracht, dass die Querbohrungen des äußeren Elements und des inneren Elements fluchten. In dieser Position wird der Faden sowohl durch die Querbohrungen des inneren als auch des äußeren Elements durchgefädelt. Dann wird der Faden durch axiales Verschieben des äußeren Elements relativ zu dem inneren Element zwischen dem inneren und dem äußeren Element geklemmt.

Aus der US 5,464,427 ist ein spreizbarer Fadenanker bekannt. Der aus diesem Dokument bekannte Fadenanker besteht aus einem im Allgemeinen zylindrischen Grundkörper und einem annähernd konischen Körper. Der annähernd konische Körper ist mit seinem sich verjüngenden Ende in einen der Länge nach verlaufenden Schlitz des Grundkörpers einführbar, wodurch eine Spreizung des letzterem bewirkt wird. Die Spreizung des Nahtankers führt zu einer verbesserten Fixierung des Ankers in der Knochenmasse.

Aus der eingangs genannten US 2005/0245932 A1 sind Ankerelemente bekannt, die zum knotenfreien Fixieren von Gewebe an einem Knochen mittels eines durch das Ankerelement durchgefädelten Fadens dienen. Die bekannten, insbesondere in Fig. 14 A, B bis 17 A, B dargestellten Ankerelemente weisen jeweils eine durch das Ankerelement hindurchreichende Querbohrung auf, die zum Durchfädeln zumindest eines chirurgischen Fadens quer durch das Ankerelement dient. Ferner ist bei den bekannten Ankerelementen ein etwa scheibenförmiges Klemmelement vorgesehen, das zum Klemmen des Fadens, der durch die Querbohrung durchgefädelt ist, dient. Um den Faden in der Querbohrung des Ankerelements zu klemmen, wird das scheibenförmige Klemmelement in eine Längsbohrung in dem Ankerelement, die in der Querbohrung mündet, eingetrieben und in Richtung der Querbohrung bewegt.

Dieses Dokument offenbart den Oberbegriff des Anspruchs 1.

Es ist Aufgabe der vorliegenden Erfindung, ein Ankerelement der eingangs genannten Art zu schaffen, das einfach aufgebaut ist, eine wirksame Verankerung des Ankerelements sicherstellt und zugleich eine wirksame und schadensfreie Fixierung des Fadens ermöglicht.

Erfindungsgemäß wird die Aufgabe zum einen dadurch gelöst, dass das gesamte innerhalb des Körpers angeordnete Klemmelement als Stift ausgebildet ist, der längs einer Längsachse des Körpers in Richtung auf die Querbohrung zu und in diese hineinbewegbar ist, und dass das Klemmelement über ein Gewinde im Körper fixierbar ist.

Erfindungsgemäß wird die Aufgabe zum anderen dadurch gelöst, dass das gesamte innerhalb des Körpers angeordnete Klemmelement als Stift ausgebildet ist, der längs einer Längsachse des Körpers in Richtung auf die Querbohrung zu und in diese hineinbewegbar ist, und dass das Klemmelement als konisches Element ausgebildet ist.

Ein erheblicher Vorteil der Maßnahme der Ausbildung des innerhalb des Körpers angeordneten Abschnitts des Ankerelements als Stift besteht darin, dass diese Geometrie ein kompaktes, stabiles Klemmelement erlaubt, das, auch bei insgesamt sehr geringen Baugrößen des Ankerelements, die notwendige Klemmkraft übertragen kann.

Die Maßnahme des Anordnens eines Abschnittes des verschiebbaren Klemmelements im Inneren des Körpers hat den Vorteil, dass die Außenseite des Körpers durch dieses Klemmelement nicht belegt ist, so dass die Außenseite des Körpers des Klemmelements voll zur Verankerung mit dem Knochen zur Verfügung steht. Ist der im Inneren angeordnete Abschnitt des Klemmelements von der Querbohrung wegbewegt, kann der durch die Querbohrung hindurchgefädelte Faden frei bewegt werden. Durch das Bewegen des inneren Abschnittes des Klemmelements in Richtung der Querbohrung kann nunmehr der in der Querbohrung aufgenommene Abschnitt des Fadens geklemmt werden.

Dies kann durch das Klemmelement selbst unmittelbar erfolgen oder das Klemmelement kann ein weiteres Element, beispielsweise eine in der Querbohrung aufgenommene Klemmhilfe, quetschen. Da bei diesen Bewegungen die Außenseite des Ankerelementes nicht belegt werden muss, können diese Manipulationen im Inneren des Körpers durchgeführt werden, nachdem bereits das Ankerelement im Knochen eingetrieben ist. Dadurch sind beispielsweise auch Korrekturen des Fadensitzes bei bereits eingebrachtem Ankerelement möglich, ohne dass dazu das Ankerelement als solches relativ zum Knochen bewegt werden muss.

Die Handhabung des Klemmelements im Inneren des Körpers kann von der proximalen Seite des Ankerelementes her bewerkstelligt werden, wozu das Klemmelement auch proximalseitig vorragen könnte. Der Körper des Ankerelementes kann, was seine äußeren Konstruktionsmerkmale betrifft, genauso aufgebaut sein, wie ein Ankerelement, das mit der Verknotung arbeitet. Es muss lediglich im Inneren des Körpers eine entsprechende Aushöhlung oder Bohrung vorgesehen sein, die sehr einfach zu bewerkstelligen ist, in die der innenliegende Abschnitt des Klemmelements eingebracht werden kann. Dieser innere Abschnitt kann dann entsprechend massiv ausgebildet sein, um die ausreichende Klemmkraft auf den in der Querbohrung aufgenommenen Faden ausüben zu können.

Als Widerlager für die Klemmung durch das Klemmelement steht die ohnehin vorhandene innere Wand der Querbohrung zur Verfügung. Diese Wand stellt dem Faden eine ausreichend große Anlagefläche zur Verfügung, an der der zu klemmende Faden durch das verschobene Klemmelement angelegt bzw. fixiert werden kann. In anderen Worten ausgedrückt kann sich der Faden beim Verklemmen an die Innenwand der Querbohrung anlegen, so dass die Klemmkräfte über einen relativ großen Flächenbereich verteilt werden, so dass die Gefahr von Verletzungen und Abscheren des Fadens stark herabgesetzt ist.

Die Maßnahme, dass das Klemmelement innerhalb des Körpers angeordnet ist, zeigt den Vorteil, dass die Außenkontur des Ankerelements in keinster Weise durch das Vorsehen des Klemmelementes beeinträchtigt wird, das heißt die ganze Außenkontur des Ankerelements kann zur eigentlichen Funktion, nämlich zur Verankerung im Knochen, herangezogen werden. Von außen betrachtet kann ein erfindungsgemäßes Ankerelement konstruktiv gleich aussehen, wie ein beispielsweise aus der US 5,690,676 bekanntes Ankerelement, das bei der Technik mit der Bewerkstellung der Verknotung eingesetzt wird. Hier bestehen schon ausreichende Erfahrungen über die konstruktiven Ausgestaltungen, um eine ausreichende Verankerungskraft bereitzustellen, so dass auf diesen Erfahrungsschatz zurückgegriffen werden.

Die Maßnahme, dass das Klemmelement als konisches Element ausgebildet ist, hat den Vorteil, dass ein solches Element sehr einfach herzustellen ist und zum Fixieren des Fadens lediglich im Inneren des Körpers vorgetrieben werden muss. Durch entsprechende Auswahl des Konuswinkels und der Länge des inneren Abschnittes kann die ausreichende Haltekraft bzw. Klemmkraft ausgeübt werden.

Somit wird die Aufgabe vollkommen gelöst.

In einer weiteren Ausgestaltung der Erfindung ist der Stift als Madenschraube ausgebildet.

Diese Maßnahme hat den Vorteil, dass ein sehr kompaktes und einfach zu steuerndes Klemmelement vorhanden ist, das durch einfaches Verdrehen entsprechend im Körper des Ankerelementes vor- und zurückbewegt werden kann.

In einer weiteren Ausgestaltung der Erfindung ist ein distales Ende des Klemmelementes abgerundet.

Diese Maßnahme hat den Vorteil, dass das zuvor erwähnte Andrücken oder Anpressen des Fadens in der Queröffnung scherkraftarm und ohne Verletzung des Fadens ausgeübt werden kann.

In einer weiteren Ausgestaltung der Erfindung weist das Klemmelement ein Montagemerkmal auf, an das ein Werkzeug eines Eintreibers für das Ankerelement ansetzbar ist.

Diese Maßnahme hat nun den erheblichen Vorteil, dass mit demselben Werkzeug, mit dem das Ankerelement eingetrieben wird, auch zugleich das Klemmelement gesteuert werden kann, sprich zum Klemmen des Fadens in Richtung der Querbohrung bewegt werden kann.

Diese Maßnahme hat in fertigungs- und handhabungstechnischer Hinsicht den Vorteil, dass nicht zwei Werkzeuge bereitgestellt werden müssen, eines zum Eintreiben des Ankers und eines zum Bewegen des Klemmelementes. Das Setzen des Ankerelementes und das Verschieben des Klemmelementes können in einem Arbeitsvorgang mit einem Werkzeug nacheinander durchgeführt werden.

Dies ist für den Operateur besonders hilfreich und fördert diese Operationstechnik.

Die für die Bauelemente eingesetzten Materialien können je nach Wunsch metallische Materialien, insbesondere Titan, sein, oder diese können aus resorbierbaren Materialien hergestellt sein, falls dies gewünscht wird.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele im Zusammenhang mit den beiliegenden Zeichnungen näher beschrieben und erläutert.

Es zeigen:
- Figur 1: eine Explosionsdarstellung eines erfindungsgemäßen Ankerelements und dessen Klemmelements, wobei am unteren Ende das Handhabungswerkzeug zum Setzen des Ankerelementes in den Knochen dargestellt ist,
- Figur 2: einen Längsschnitt des Ankerelementes von Figur 1 mit eingesetztem Klemmelement, wobei das Handhabungswerkzeug angesetzt ist und ein Faden durch das Ankerelement hindurchgefädelt ist, der aber noch frei bewegbar ist,
- Figur 3: eine Momentaufnahme der Operationstechnik zum Setzen des Ankerelementes, wobei der in Figur 2 dargestellte Zusammenbau ersichtlich ist, der durchgezogene Faden bereits mit einem Gewebe verbunden und wieder durch die Querbohrung hindurchgezogen wurde,
- Figur 4: einen Schnitt nach Setzen des Ankerelementes und Fixieren der Sehne mittels des Fadens und Fixieren des Fadens mittels des Klemmelementes in dem Knochen, und
- Figur 5: eine Explosionsdarstellung eines weiteren Ausführungsbeispiels eines erfindungsgemäßen Ankerelementes mit keilförmigem Klemmelement.

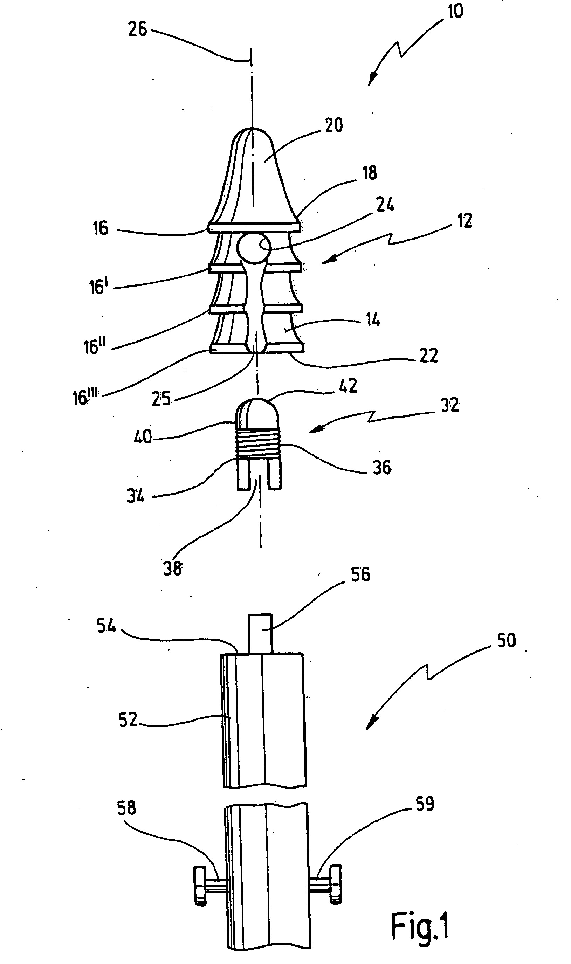

Ein in den Figuren 1 bis 4 dargestelltes Ankerelement ist in seiner Gesamtheit mit der Bezugsziffer 10 bezeichnet.

Das Ankerelement 10 weist einen langerstreckten grob zylindrischen Körper 12 auf, an dessen Außenseite 14 mehrere Vorsprünge 16 bis 16"' vorspringen. Die Vorsprünge 16 bis 16"' sind als vorspringende Ringflansche ausgebildet, die jeweils nach distal gesehen über etwa konisch verjüngende Abschnitte zum nächsten Ringflansch übergehen. Ein distaler Endbereich 18 des Körpers 12 ist mit einer abgerundeten Spitze 20 versehen. Ein proximales Ende 22 wird durch die Querschnittsfläche des letzten Vorsprunges 16"' gebildet.

Im distalen Endbereich 18 des Körpers 12 ist eine durch diesen hindurchgehende Querbohrung 24 angeordnet. Von der Mündungsöffnung der Querbohrung 24 reichen längs der Längsachse 26 des Körpers 12 zwei diametral gegenüberliegende Längsnuten 25, die in die Vorsprüngen 16' bis 16"' eingeschnitten sind.

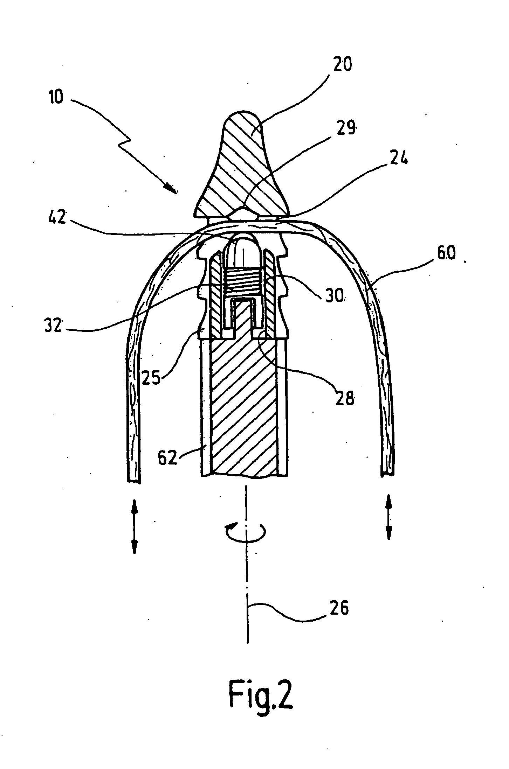

Wie an sich bekannt, dient die Querbohrung dazu, um einen Faden 60, wie das in Figur 2 dargestellt ist, durch den Körper 12 hindurchzufädeln. Die beidseits der Querbohrung 24 vorstehenden Fadenabschnitte können in die Längsnuten 25 eingelegt werden, so dass diese Fadenabschnitte eng am Körper 12 anliegend von der Querbohrung 24 kommend nach proximal geführt werden können.

Mittig im Körper 12 ist von proximal eine axiale Bohrung 28 eingebracht (siehe Figur 2), die distalseitig in die Querbohrung 24 mündet.

In der axialen Bohrung 28 ist ein Innengewinde 30 eingeschnitten.

Diese axiale Bohrung 28 dient zur Aufnahme eines Klemmelementes 32.

Das Klemmelement 32 besteht aus einem Stift 34, an dessen Außenseite ein Außengewinde 36 vorhanden ist, das mit dem Innengewinde 30 der axialen Bohrung 28 kämmt.

Am proximalen Ende ist im Stift 34 eine Einsenkung 38, hier in Form eines diametralen Einschnittes vorgesehen, deren Sinn und Zweck nachfolgend erläutert wird. Am distalen Ende 40 ist das Klemmelement 33 mit einer Rundung 42 versehen.

In Figur 1 ist am unteren Ende ein sogenannter Eintreiber 50 dargestellt, mit dem das Ankerelement 10 gehandhabt wird.

Der Eintreiber 50 weist einen Stab 52 auf, von dessen distaler Stirnseite 52 ein Werkzeug 56 vorspringt, das so ausgebildet ist, dass es formschlüssig in die Einsenkung 38 des Klemmelements 32 eingesetzt werden kann. Die Stirnseite 54 des Eintreibers 50 ist ferner so ausgebildet, dass diese am proximalen Ende 22 des Körpers 12 des Ankerelements 10 angelegt werden kann.

In axialem Abstand zu seiner Stirnseite 54 ist der Eintreiber 50 mit zwei diametral vorstehenden Zapfen 58, 59 versehen, die dazu dienen, um die beiden vorstehenden Fadenenden aufzuwickeln. Proximalseitig endet der Stab 52 in einem hier nicht dargestellten Handgriff, über den der Eintreiber 50 von der Handhabungsperson von Hand ergriffen werden kann.

In Figur 2 ist nun eine Situation dargestellt, in der das Klemmelement 32 im Inneren des Körpers 12 aufgenommen ist, dabei ist das Außengewinde 36 in das Innengewinde 30 eingedreht, und zwar soweit, dass der durch die Querbohrung 24 durchgeführte Faden 60 frei bewegbar ist, wie das durch die Doppelpfeile angedeutet ist.

Das proximale Ende 22 des Körpers 12 sitzt auf der Stirnseite 54 des Eintreibers 50, dessen Werkzeug 56 greift dabei in die Einsenkung 38 des Klemmelementes 32 ein.

Wie zuvor erwähnt dienen die Längsnuten 25 an der Außenseite 14 des Körpers 12 dazu, die Fadenenden eng angeschmiegt nach proximal zu führen. Dem entsprechend sind in dem Stab 52 des Eintreibers 50 Nuten 62 eingeschnitten, um diese Fadenenden bis zu den diametral vorstehenden Zapfen 58, 59 zu führen, um die diese gewickelt werden.

Wie insbesondere aus der Schnittdarstellung von Figur 2 ersichtlich, bewirkt ein Drehen des Eintreibers 50 um die Längsachse 26 des Zusammenbaus ein Drehen des Klemmelements 32 im Inneren des Ankerelementes 10, wodurch das Klemmelement 32 in Richtung auf die Querbohrung 24 zu und in diese hineinbewegt wird. Dabei wird der in der Querbohrung 24 aufgenommene Abschnitt des Fadens 60 durch die Rundung 42 an die dieser gegenüberliegende innere Wand der Querbohrung 24 gelegt und bei weiterem Vortreiben entsprechend geklemmt.

Durch die runde und sanfte Konturierung von Rundung 42 des Klemmelementes 32 und der entsprechenden Konturierung der Innenwand der Querbohrung 24 kann die Klemmkraft auf den Faden über einen relativ großen Flächenbereich verteilt werden, wodurch ein Abquetschen oder Abscheren des fixierten Fadens 60 in der Querbohrung 24 vermieden werden kann.

Aus der Schnittdarstellung von Figur 2 ist ersichtlich, dass die axiale Bohrung 28 distalseitig der Querbohrung 24 in einer Art sanften Mulde 29 weitergeführt ist, die in etwa der Kontur der Rundung 42 entspricht, so dass der Faden 60 besonders schonend, jedoch fest zwischen Mulde 29 und Rundung 42 fixiert werden kann.

Wie aus Figur 2 zu entnehmen, kann das an der Stirnseite 54 des Eintreibers 50 anliegende Ankerelement 10 mittels diesem in einen Knochen eingetrieben bzw. eingeschlagen werden.

Die Länge des Werkzeuges 56 bzw. die Tiefe der Einsenkung 38 im Klemmelement 32 sind so gewählt, dass dabei keine Beeinträchtigung des Klemmelementes 32 stattfindet, ein ausreichender Eingriff zwischen diesen beiden Bauelementen jedoch gewährleistet ist, um ein nachfolgendes Drehen des Klemmelementes 32 zum Bewerkstelligen des Klemmvorgangs bewirken zu können.

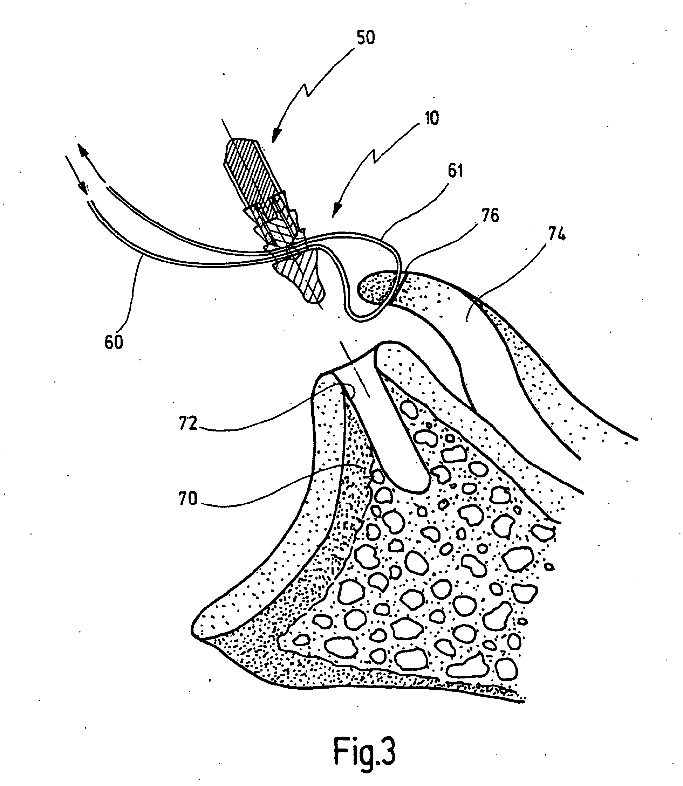

In Figur 3 ist eine Momentaufnahme einer Operationstechnik, bei der ein erfindungsgemäßes Ankerelement 10 eingesetzt wird, dargestellt.

Von einem Knochen 70 hat sich ein Teil eines Gewebes 74, beispielsweise eine Sehne, abgelöst, die wieder am Knochen 70 fixiert werden soll.

Im dargestellten Ausführungsbeispiel wurde im Bereich der Ablösung in den Knochen 70 eine Öffnung 72, beispielsweise eine Bohrung, bewerkstelligt, deren Innendurchmesser etwas geringer ist als der Außendurchmesser der Vorsprünge 16 des Körpers 12 des Ankerelementes 10. Der Zusammenbau aus Ankerelement 10 mit darin aufgenommenem Klemmelement 32 und Eintreiber 50 wird an die Operationsstelle verbracht, und der Faden 60 wird wie in Figur 2 dargestellt einmal durch die Querbohrung 24 hindurchgefädelt. Eines der freien Enden wird, ggf. unter Zuhilfenahme einer Nadel, durch einen abgelösten Abschnitt des Gewebes 74 hindurchgestochen, wobei in Figur 3 dieser Durchstich 76 im Schnitt ersichtlich ist. Der durch den Durchstich 76 austretende Fadenabschnitt wird erneut durch die Querbohrung 24 hindurchgeführt, und zwar in entgegengesetzter Richtung wie zuvor.

Dadurch entsteht, wie das aus Figur 3 ersichtlich ist, eine Schleife 61, über die das Gewebe 74 mit dem Ankerelement 10 verbunden ist. Die beiden freien Fadenenden werden nunmehr längs des Zusammenbaus aus Eintreiber 50 und Ankerelement 10 eng anliegend zu den Zapfen 58, 59 geführt, und um diese gefädelt.

Anschließend wird das Ankerelement 10 in die Öffnung 72 des Knochens 70 mittels des Eintreibers 50 eingeschlagen. Durch Ziehen an den freien Enden des Fadens 60 kann der losgelöste Gewebeabschnitt 74 in die gewünschte Relativposition zum Knochen bzw. zum Ankerelement gebracht werden. Durch Drehen des Eintreibers 50 wird nunmehr das Klemmelement 32 in die Querbohrung 24 hineinbewegt und klemmt die beiden in der Querbohrung aufgenommenen Fadenabschnitte. Der Eintreiber 50 wird abgenommen und die überstehenden Fadenabschnitte können abgetrennt werden.

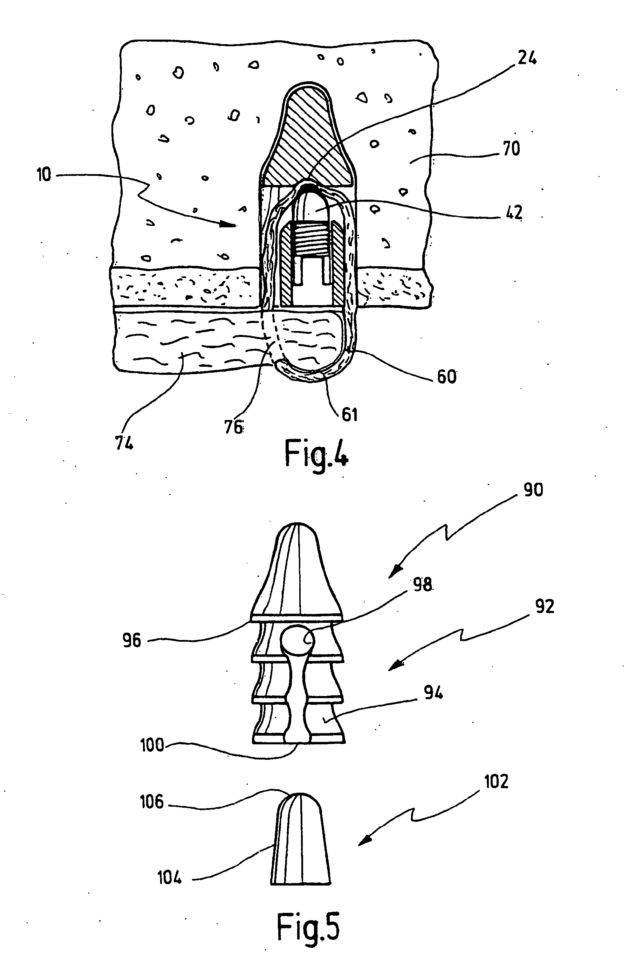

Hier ist es durchaus noch möglich, gewisse Korrekturen des Fadensitzes auch bei schon gesetztem Ankerelement 10 durchzuführen, indem nochmals das Klemmelement 32 etwas gelockert wird. Nach Abziehen des Eintreibers 50 liegt das zu fixierende Gewebe 74, wie das in Figur 4 dargestellt ist, wieder am Knochen 70 an. Lediglich die Schleife 61 des Fadens 60 ist ersichtlich, somit ist kein bulkiger Knotenabschnitt vorhanden. Im Inneren des Körpers 12 sind die in der Querbohrung 24 aufgenommenen Abschnitte des Fadens 16 durch das eingedrehte Klemmelement 32 lagefixiert.

In Figur 5 ist ein weiteres Ausführungsbeispiel eines Ankerelementes dargestellt, das in seiner Gesamtheit mit der Bezugsziffer 90 bezeichnet ist. Auch dieses Ankerelement 90 weist einen Körper 92 auf, an dessen Außenseite 94 entsprechende Vorsprünge 96 ausgebildet sind. Auch hier ist eine Querbohrung 98 zur Durchführung des Fadens vorgesehen.

Es ist in dem Vergleich zu Figur 1 ersichtlich, dass die Außenkontur des Körpers 92 gleich ausgebildet ist, wie die Außenkontur des Körpers 12.

Die axiale Bohrung 100 im Inneren des Körpers 92 ist als konische Bohrung ausgeführt, in die ein Klemmelement 102 eingeführt werden kann, das einen entsprechenden Konus 104 aufweist.

Auch hier weist das Klemmelement 102 wieder ein abgerundetes distales Ende 106 auf.

Die Technik des Setzens des Ankerelementes 90 ist vergleichbar mit der zuvor beschriebenen Technik. Zum Klemmen des Fadens wird allerdings hier das Klemmelement 102 in die axiale Bohrung 100 mittels des Eintreibers eingeschlagen und bewerkstelligt dadurch das Klemmen des Fadens.

Der Konuswinkel ist entsprechend klein gewählt, so dass kein Selbstlöseeffekt, sondern der ausreichende Klemm- oder Hemmeffekt bei eingeschlagenen Klemmelement 102 sichergestellt ist.

Das Ankerelement 90 kann beispielsweise aus bioresorbierbaren Materialien hergestellt sein, was auch für das Ankerelement 10 gelten kann.

Das Ankerelement 10 kann auch aus metallischem Material, beispielsweise aus Titan, hergestellt werden.

Die Länge solcher Ankerelemente liegt üblicherweise im Bereich zwischen 3 und 6 Zentimeter, der Durchmesser liegt im Bereich von einigen Millimetern bis in den Bereich von etwa einem Zentimeter. Auch bei nichtmetallischen Materialien ist eine ausreichende mechanische Stabilität vorhanden, sowohl was die Verankerung im Knochen als auch was die Fixierung des im Ankerelement durchgefädelten Fadens betrifft.

## Patentansprüche

1. Ankerelement zum knotenfreien Fixieren von Gewebe (74) an einem Knochen (70) mittels zumindest eines durch das Ankerelement (10) hindurchgefädelten Fadens (60), mit einem Körper (12), an dessen Außenseite (14) Vorsprünge (16 bis 16"') vorgesehen sind, die ein Abziehen des im Knochen (70) eingebrachten Ankerelementes (10) hindern, mit einer im distalen Endbereich (18) des Körpers (12) angeordneten und durch diesen hindurchreichenden Querbohrung (24) zum Durchfädeln des zumindest einen Fadens (60) quer durch den Körper (12) hindurch, und mit einem längs des Körpers (12) bewegbaren Klemmelements (32) zum Klemmen des durch den Körper (12) hindurchgefädelten Fadens (60), wobei das Klemmelement (32) innerhalb des Körpers (12) angeordnet ist und zum Klemmen des Fadens (60) in Richtung der Querbohrung (24) bewegbar ist, **dadurch gekennzeichnet, dass** das gesamte innerhalb des Körpers (12) angeordnete Klemmelement (32) als Stift (34) ausgebildet ist, der längs einer Längsachse (26) des Körpers (12) in Richtung auf die Querbohrung (24) zu und in diese hineinbewegbar ist, und dass das Klemmelement (32) über ein Außengewinde (36) im Körper (12) fixierbar ist.

2. Ankerelement zum knotenfreien Fixieren von Gewebe (74) an einem Knochen (70) mittels zumindest eines durch das Ankerelement (90) hindurchgefädelten Fadens (60), mit einem Körper (92), an dessen Außenseite (94) Vorsprünge (96) vorgesehen sind, die ein Abziehen des im Knochen (70) eingebrachten Ankerelementes (90) hindern, mit einer im distalen Endbereich (18) des Körpers (92) angeordneten und durch diesen hindurchreichenden Querbohrung (98) zum Durchfädeln des zumindest einen Fadens (60) quer durch den Körper (92) hindurch, und mit einem längs des Körpers (92) bewegbaren Klemmelements (102) zum Klemmen des durch den Körper (92) hindurchgefädelten Fadens (60), wobei das Klemmelement (102) innerhalb des Körpers (92) angeordnet ist und zum Klemmen des Fadens (60) in Richtung der Querbohrung (98) bewegbar ist, **dadurch gekennzeichnet, dass** das gesamte innerhalb des Körpers (92) angeordnete Klemmelement (102) als Stift (34) ausgebildet ist, der längs einer Längsachse (26) des Körpers (92) in Richtung auf die Querbohrung (98) zu und in diese hineinbewegbar ist, und dass das Klemmelement (102) als konisches Element (104) ausgebildet ist.

3. Ankerelement nach Anspruch 1, **dadurch gekennzeichnet, dass** der Stift (34) als Madenschraube ausgebildet ist.

4. Ankerelement nach einem der Ansprüche 1 bis 3, **dadurch gekennzeichnet, dass** ein distales Ende (40, 106) des Klemmelementes (32, 102) abgerundet ist.

5. Ankerelement nach einem der Ansprüche 1 bis 4, **dadurch gekennzeichnet, dass** das Klemmelement (32, 102) ein Montagemerkmal (38) aufweist, an das ein Werkzeug (56) eines Eintreibers (50) für das Ankerelement (10, 90) ansetzbar ist.

## Claims

1. An anchor element for knot-less fixing of a tissue (74) to a bone (70) by means of at least one suture (60) threaded through said anchor element (10), comprising a body (12) having projections (16 to 16"') projecting from its outer face (14) for preventing removal of said anchor element (10) when inserted in the bone (70), a transverse bore (24) arranged in a distal end section (18) of said body (12), said transverse bore (24) extending through said body (12), and serving for threading said at least one suture (60) transversely through said body (12), and comprising a clamping element (32) for clamping said suture (60) threaded through said body (12), wherein said clamping element (32) is arranged within said body (12), and is movable towards said transverse bore (24) for clamping said suture (60), **characterized in that** the whole clamping element (32) arranged inside said body (12) is designed as a pin (34) which is movable along a longitudinal axis (26) of said body (12) towards and into said transverse bore (24), and that the clamping element (32) is fixable via an external thread (36) in said body (12).

2. An anchor element for knot-less fixing of a tissue (74) to a bone (70) by means of at least one suture (60) threaded through said anchor element (90), comprising a body (92) having projections (96) projecting from its outer face (94) for preventing removal of said anchor element (90) when inserted in the bone (70), a transverse bore (98) arranged in a distal end section (18) of said body (92), said transverse bore (98) extending through said body (92), and serving for threading said at least one suture (60) transversely through said body (92), and comprising a clamping element (102) for clamping said suture (60) threaded through said body (92), wherein said clamping element (102) is arranged within said body (92), and is movable towards said transverse bore (98) for clamping said suture (60), **characterized in that** the whole clamping element (102) arranged inside said body (92) is designed as a pin (34) which is movable along a longitudinal axis (26) of said body (92) towards and into said transverse bore (98), and that the clamping element (102) is designed as a conical element (104).

3. The anchor element of claim 1, **characterized in that** said pin (34) is designed as a headless screw.

4. The anchor element of claims 1 to 3, **characterized in that** a distal end (40, 106) of said clamping element (32, 102) is rounded.

5. The anchor element of claims 1 to 4, **characterized in that** said clamping element (32, 102) comprises an assembly feature (38) onto which a tool (56) of a driver device (50) for said anchor element (10, 90) can be attached.

## Revendications

1. Élément d'ancrage pour la fixation sans noeud d'un tissu (74) sur un os (70) au moyen d'au moins un fil (60) enfilé à travers l'élément d'ancrage (10), avec un corps (12) sur la face extérieure (14) duquel sont prévues des saillies (16 à 16"') qui empêchent l'extraction de l'élément d'ancrage (10) inséré dans l'os (70), avec un perçage transversal (24) disposé dans la région terminale distale (18) du corps (12) en traversant le corps (12) de part en part et destiné à enfiler transversalement le fil au moins unique (60) à travers le corps (12), et avec un élément de serrage (32) pouvant être déplacé le long du corps (12) et destiné à serrer le fil (60) enfilé à travers le corps (12), sachant que l'élément de serrage (32) est disposé à l'intérieur du corps (12) et peut être déplacé en direction du perçage transversal (24) pour serrer le fil (60), **caractérisé en ce que** l'élément de serrage (32) disposé à l'intérieur du corps (12) est réalisé dans sa totalité sous forme de broche (34) qui peut être déplacée le long d'un axe longitudinal (26) du corps (12) en direction du perçage transversal (24) et à l'intérieur de ce dernier, et **en ce que** l'élément de serrage (32) peut être fixé dans le corps (12) au moyen d'un filetage extérieur (36).

2. Élément d'ancrage pour la fixation sans noeud d'un tissu (74) sur un os (70) au moyen d'au moins un fil (60) enfilé à travers l'élément d'ancrage (90), avec un corps (92) sur la face extérieure (94) duquel sont prévues des saillies (96) qui empêchent l'extraction de l'élément d'ancrage (90) inséré dans l'os (70), avec un perçage transversal (98) disposé dans la région terminale distale (18) du corps (92) en traversant le corps (92) de part en part et destiné à enfiler transversalement le fil au moins unique (60) à travers le corps (92), et avec un élément de serrage (102) pouvant être déplacé le long du corps (92) et destiné à serrer le fil (60) enfilé à travers le corps (92), sachant que l'élément de serrage (102) est disposé à l'intérieur du corps (92) et peut être déplacé en direction du perçage transversal (98) pour serrer le fil (60), **caractérisé en ce que** l'élément de serrage (102) disposé à l'intérieur du corps (92) est réalisé dans sa totalité sous forme de broche (34) qui peut être déplacée le long d'un axe longitudinal (26) du corps (92) en direction du perçage transversal (98) et à l'intérieur de ce dernier, et **en ce que** l'élément de serrage (102) est réalisé sous la forme d'un élément conique (104).

3. Élément d'ancrage selon la revendication 1, **caractérisé en ce que** la broche (34) est réalisée sous la forme d'une vis sans tête.

4. Élément d'ancrage selon l'une des revendications 1 à 3, **caractérisé en ce qu'**une extrémité distale (40, 106) de l'élément de serrage (32, 102) est arrondie.

5. Élément d'ancrage selon l'une des revendications 1 à 4, **caractérisé en ce que** l'élément de serrage (32, 102) présente un attribut de montage (38) sur lequel peut être appliqué un outil (56) d'un poussoir (50) pour l'élément d'ancrage (10, 90).
